# EUROPÄISCHE PATENTANMELDUNG

(11) **EP 1 654 982 A1**
(43) Veröffentlichungstag der Anmeldung: **10.05.2006**
(21) Anmeldenummer: 05110133.5
(22) Anmeldetag: 28.10.2005
(51) Int. Cl.: A61B 5/0484, A61B 3/113

(54) **System zur Messung und Interpretation von Hirnaktivitäten**

(30) Priorität: 08.11.2004 DE 102004053774
(71) Anmelder: SIEMENS AKTIENGESELLSCHAFT, 80333 München (DE)
(72) Erfinder: Dinges, Clemens, 90587 Obermichelbach (DE); Schlereth, Michael, 91452 Wilhermsdorf (DE)

(57) **Zusammenfassung**

Die Erfindung betrifft ein System und ein Verfahren zur Erfassung und Bewertung menschlicher Gehirnaktivitäten eines Anwenders im Zusammenhang einer Umgebung des Anwenders. Mit einem Trackingsystem wird eine aktuelle Position des Anwenders in Bezug auf seine Umgebung erfasst. Mit einem Umgebungsmodell wird die reale Umgebung des Anwenders soweit modelliert, dass das System Objekte und Szenarien im Umfeld des Anwenders erkennen kann. Die Hirnaktivitäten werden gemessen und den identifizierten realen Objekten bzw. Szenarien zugeordnet, so dass das System eine Korrelation zwischen der den Anwender aktuell umgebenden Realität und seinen Hirnaktivitäten herstellen kann.

## Beschreibung

Die Erfindung betrifft ein System und ein Verfahren zur Messung und Interpretation von Hirnaktivitäten eines Anwenders.

Ein derartiges System bzw. Verfahren wird beispielsweise zur Ermittlung menschlicher Emotionen in einem bestimmten Umfeld oder Szenario verwendet. Ferner kann ein derartiges System bzw. Verfahren zur Steuerung eines technischen Gerätes, insbesondere eines Computers mit der Kraft menschlicher Gedanken eingesetzt werden.

Eine Vorrichtung zum Messen und Analysieren elektrischer Hirnströme ist beispielsweise aus DE 197 25 214 C1 bekannt, wobei die gemessenen Hirnsströme zur Ermittlung eines Hirnfunktionsindexes digitalisiert und verarbeitet werden, der Auskunft über die Normalität der Hirnfunktion eines Patienten gibt.

Ferner sind Verfahren bekannt, die Aktivitäten des Gehirns elektrisch über eine Elektroenzephalogramm (EEG) oder indirekt über eine Beobachtung der Pupilleweite zu messen und den gemessenen Signalen Bedeutungen zuzuordnen ("Sie müssen sich entspannen", Die Zeit vom 08.07.2004). Bei derartigen Verfahren wird zunächst ein Szenario festgelegt, in dessen Kontext die Gehirnaktivitäten gemessen und interpretiert werden.

Der Erfindung liegt die Aufgabe zu Grunde, ein möglichst flexibles System zur Messung und Interpretation von Hirnaktivitäten für einen Anwender in einem dynamischen Umfeld bereitzustellen.

Diese Aufgabe wird gelöst durch ein System mit
- einem Messsystem zur Erfassung von Hirnaktivitäten eines Anwenders,
- einem Speicher mit einem Umgebungsmodell einer realen Umgebung des Anwenders,
- einem Trackingsystem zur Ermittlung von in einem aktuellen Wahrnehmungsbereich des Anwenders befindlichen Objekten und/oder Szenarien in der Umgebung des Anwenders, wobei eine Identifikation der Objekte und/oder Szenarien auf Basis des Umgebungsmodells vorgesehen ist,
- und mit einer Verarbeitungseinheit zur Interpretation der Hirnaktivitäten auf Basis einer Korrelation der Hirnaktivitäten mit den im aktuellen Wahrnehmungsbereich des Anwenders befindlichen Objekten und/oder Szenarien.

Die Aufgabe wird weiter gelöst durch ein Verfahren, bei dem mit
- einem Messsystem Hirnaktivitäten eines Anwenders erfasst werden,
- einem Trackingsystem in einem aktuellen Wahrnehmungsbereich des Anwenders befindliche Objekte und/oder Szenarien in der Umgebung des Anwenders ermittelt werden, wobei die Objekte und/oder Szenarien auf Basis eines Umgebungsmodells identifiziert werden,
- und bei dem mit einer Verarbeitungseinheit die Hirnaktivitäten auf Basis einer Korrelation der Hirnaktivitäten mit den im aktuellen Wahrnehmungsbereich des Anwenders befindlichen Objekten und/oder Szenarien interpretiert werden.

Eine Interpretation der Hirnaktivitäten des Anwenders erfordert Kenntnis über die Szenarien oder Objekte, die Auslöser der gemessenen Hirnaktivitäten sind. Das erfindungsgemäße System ermöglicht die Interpretation der Hirnaktivitäten des Anwenders sogar im Kontext wechselnder Szenarien durch die Kombination des Messsystems zur Erfassung der Hirnaktivitäten mit dem Trackingsystem.

Das Trackingsystem ermöglicht es, die Szenarien oder Objekte in der Umgebung des Anwenders zu identifizieren, die den Anwender aktuell interessieren. Hierzu wird das Umgebungsmodell benötigt, in dem die Umgebung des Anwenders soweit modelliert ist, wie es zu Erkennung von Objekten und damit von Szenarien notwendig ist.

Zunächst wird mit Hilfe des Trackingsystems der Bereich der Umgebung identifiziert, auf das sich der Fokus des Anwenderinteresses richtet. Dies kann beispielsweise durch eine Erfassung der Position und des Blickwinkels des Anwenders erfolgen. Anhand des im Speicher abgelegten Umgebungsmodells kann das System die Objekte und Szenarien identifizieren, die gemäß des erfassten Blickwinkels und der Position des Anwenders im Wahrnehmungsbereich des Anwenders liegen. Durch das parallele Erfassen der Hirnaktivitäten können die erfassten Emotionen den entsprechenden Objekten bzw. Szenarien zugeordnet werden.

Im Gegensatz zu bekannten Systemen zur Ermittlung und Interpretation der Emotionen eines Menschen muss bei dem erfindungsgemäßen System das Szenario, in dem sich der Mensch bei der Messung der Hirnaktivitäten befindet, nicht im Vorhinein festgelegt werden. Vielmehr erlaubt die Erfindung eine Messung und eine Interpretation der Hirnaktivitäten bei verschiedenen wechselnden Szenarien, indem diese mit Hilfe des Trackingsystems und des Umgebungsmodells dynamisch erkannt werden. Welche Objekte/Szenarien mit dem Trackingsystem erkannt werden können, ist in erster Linie vom Umfang des Umgebungsmodells bestimmt.

In einer vorteilhaften Ausführungsform der Erfindung ist die Verarbeitungseinheit zur Erzeugung einer Datenmenge vorgesehen, die das Ergebnis der Interpretation beschreibt. Die erzeugte Datenmenge kann einem weiteren Verarbeitungssystem gesendet werden, welches in Abhängigkeit der Datenmenge neue Handlungen auslöst.

In einer vorteilhaften Ausführungsform der Erfindung ist das System mit Sendemitteln zur insbesondere drahtlosen Übertragung der Datenmenge an das Objekt ausgeführt, wobei das Objekt als technische Vorrichtung mit einer Signalverarbeitungseinheit zur Verarbeitung der Datenmenge ausgebildet ist. Szenarien bzw. Objekte, die der Anwender über seine Sinnesorgane wahrnimmt und die eine Veränderung seiner Hirnaktivitäten auslösen, werden von dem System erkannt, interpretiert und in Form der Datenmenge an die technische Vorrichtung gesendet. Auf diese Weise ist es möglich, die menschliche Sensorik einer technischen Vorrichtung zugänglich zu machen.

Die menschliche Sensorik ist in vielen Fällen einer technischen Sensorik, wie sie heute beispielsweise in der Automatisierungstechnik eingesetzt wird, weit überlegen. Beispielhaft sei hier die visuelle Überprüfung des Reifegrades von Früchten, der Vollständigkeit von Hummern, der Freiheit von Schnitt- und Nähfehlern bei manuell in Serie gefertigten Bekleidungsstücke und der Wurmfreiheit von Meeresfischen genannt. Auch die taktilen Wahrnehmungsfähigkeiten des Menschen können mit dem System genutzt werden z. B. zur Erkennung von Lackfehlern bei Kfz-Blechen, bei der Überprüfung von entgrateten Gehäuseblechen oder auch des Reifegrades von Früchten. Für derartige sensorische Aufgaben sind die Sinnesorgane des Menschen deutlich besser geeignet als heutige Ausführungsformen technischer Sensoren. Die beschriebene Ausführungsform macht der technischen Vorrichtung eben diese menschlichen sensorischen Fähigkeiten zugänglich.

Zur Ermittlung der im aktuellen Wahrnehmungsbereich des Anwenders befindlichen Objekte und/oder Szenarien in der Umgebung des Anwenders sind verschiedene Ausführungen des Trackingsystems denkbar und von der Erfindung umfasst. Wenn eine grobe Positionsbestimmung des Anwenders ausreicht, um Rückschlüsse auf die Objekte und/oder Szenarien in seinem Umfeld zu ziehen, die im Fokus seines Interesses liegen, ist das Trackingsystem z. B. zur Bestimmung der Position des Anwenders in Bezug auf die Umgebung des Anwenders vorgesehen.

Eine genauere Erfassung der Objekte/Szenarien im Wahrnehmungsbereich des Anwenders ist möglich, wenn das Trackingsystem zur Bestimmung des Blickwinkels des Anwenders in Bezug auf die Umgebung des Anwenders vorgesehen ist. Hierzu kann insbesondere ein Eye-Trackingsystem verwendet werden, mit dem die Blickrichtung des Anwenders erfasst wird.

Bei einer weiteren alternativen Ausführungsform des erfindungsgemäßen Systems ist das Trackingsystem zur Lagebestimmung eines Körperteils des Anwenders vorgesehen. Beispielsweise kann an einem Finger des Anwenders ein Sensor befestigt sein, dessen Position von einer Erfassungseinheit verfolgbar ist. Die Position des Fingers kann alternativ auch über eine Kapazitätsmessung gegenüber einer Oberfläche ermittelt werden. Bei einer derartigen Ausführungsform wird die Position des Fingers als Indiz für das oder die Objekte angesehen, die im Fokus des Anwenderinteresses stehen.

Um im Vorhinein das Objekt bzw. Szenario aus dem Umfeld des Menschen zu ermittelt, welches aktuell dessen Interesse weckt, ist es zweckmäßig, das System mit einer Aufzeichnungseinheit zur Aufzeichnung von durch das Trackingsystem ermittelten Informationen auszuführen. Hierzu ist beispielsweise ein Ringpuffer mit einer Speicherzeit im Bereich von 2 Sekunden geeignet.

In einer besonders vorteilhaften Ausführungsform ist das System mit einem Augmented Reality System ausgeführt. Augmented Reality, erweiterte Realität, ist eine Form der Mensch-Technik-Interaktion, die dem Menschen z. B. über eine Datenbrille Informationen in sein Sichtfeld einblendet und damit die von ihm wahrgenommene aktuelle Realität erweitert. Dieses geschieht kontextabhängig, d. h. passend zum und abgeleitet vom betrachteten Objekt, z. B. einem Bauteil, einem Werkzeug, einer Maschine oder zu seinem Standort. Beispiel hierfür kann ein Sicherheitshinweis während eines Montage-/Demontageprozesses sein. Derartige Systeme sind in der Regel ebenfalls mit einem Trackingsystem ausgeführt, um eine Positionsbestimmung eines Anwenders in Bezug auf seine reale Umgebung durchführen zu können. Durch die Kombination eines Augmented Reality Systems mit dem Messsystem zur Erfassung der Hirnaktivitäten lassen sich z. B. bei der Einblendung der Informationen über die Datenbrille erfasste Gedanken bzw. Emotionen des Anwenders mit berücksichtigen.

In einer vorteilhaften Ausführungsform der Erfindung wird ein Leitfähigkeitsmesssystem zur Bestimmung der Leitfähigkeit der Haut des Anwenders verwendet. Eine derartige Messmethode zur Ermittlung des Gemütszustandes des Anwenders ist beispielsweise von Lügendetektoren bekannt. Unter bestimmten emotionalen Bedingungen, z. B. Stress, ändert sich die Zusammensetzung des Wasser-Fettfilms auf der Haut, was zu einer Veränderung der Oberflächenleitfähigkeit der Haut führt, die wiederum mittels einer Leitfähigkeitsmessung detektierbar ist.

Zur Ermittlung der Hirnaktivitäten des Anwenders ist in einer zweckmäßigen Ausführungsform das Messsystem zur Erfassung der Hirnaktivitäten zur Hirnstromessung vorgesehen. Hierzu trägt der Anwender in der Regel eine EEG Kappe (EEG = Elektroenzephalogramm), die eine Vielzahl von mit dem Kopf des Anwenders verbundenen Elektronen enthält.

Alternativ zur Messung der Hirnströme kann das Messsystem zur Erfassung der Hirnaktivitäten zur Pupillenmessung vorgesehen werden. Dieses Messverfahren beruht auf dem Effekt, dass der Irismuskel, der die Pupille verengt oder weitet, vom Hypothalamus gesteuert wird. Der Hypothalamus ist eine von den Gefühlen des Menschen beeinflusste Gehirnregion. Die Pupille gibt die Aktivität des Nervensystems sehr schnell wieder. In dieser Hinsicht ist eine Pupillenmessung zur Bestimmung des emotionalen Zustandes des Anwenders einer Leitfähigkeitsbestimmung der Haut des Anwenders überlegen.

Eine besonders vorteilhafte Verwendung einer erfindungsgemäßen Ausführungsform des Systems ist in der Automatisierungstechnik zu sehen. Durch die erfindungsgemäße Verknüpfung der menschlichen Sensorik mit einem technischen System lassen sich insbesondere in der Automatisierungstechnik erhebliche Vorteile gegenüber heutigen Formen der Mensch-Maschine-Kopplung erzielen. Gegenüber heutiger Kopplungsmechanismen wie beispielsweise der Informationsübertragung per Tastendruck an ein Automatisierungssystem oder Spracheingabe etc. ist eine verschwindet geringe Verzögerungszeit mit einer erfindungsgemäßen Ausführungsform des Systems für die Automatisierungstechnik realisierbar, da ein im Wahrnehmungsbereich des Anwenders liegendes Szenario lediglich durch die vergleichsweise geringe Reaktionszeit des menschlichen Gehirns verzögert erfasst wird. Außerdem erlaubt die erfindungsgemäße Ausführungsform eine Übermittlung der wahrgenommen Informationen ohne zur Hilfenahme der Hände des Anwenders. Somit stehen dem Anwender die Hände für weitere Aufgaben zur Verfügung.

Denkbar ist auch eine Verwendung eines Systems nach einer erfindungsgemäßen Ausführungsform zur Qualitätskontrolle in einem industriellen Fertigungsprozess. Hier kann die menschliche Sensorik u. U. für Kontrollaufgaben eingesetzt werden, die mit heutigen technischen Sensoren nicht oder nicht zufrieden stellend gelöst werden. Beispielhaft sein hier die Überprüfung einer Lackierung im Kfz-Bereich genannt.

Besonders vorteilhaft ist eine Verwendung eines Systems gemäß einer Ausführungsform der Erfindung zur Aufmerksamkeitskontrolle des Anwenders. Mit Hilfe des Trackingsystems kann z. B. eine Situation erkannt werden, die die besondere Aufmerksamkeit des Anwenders erfordert. Durch Überwachung der Hirnaktivitäten kann überprüft werden, ob der Anwender die erforderliche Aufmerksamkeit leistet. Ggf. lassen sich technische Geräte, die in einer kritische Situation involviert sind, automatisch abschalten, sobald die Aufmerksamkeit des Anwenders unzulässig nachlässt. Zur Überwachung der Aufmerksamkeit ist insbesondere auch eine Beobachtung der Augenlidfunktion des Anwenders geeignet.

Eine weitere vorteilhafte Verwendung eines Systems gemäß einer Ausführungsform der Erfindung ist die Ermittlung beabsichtigter Anwenderhandlungen an einem technischen System. Das System kann anhand der Hirnaktivitäten und der durch das Trackingsystem erfassten Informationen erkennen, dass der Anwender eine Handlung an einer Maschine beabsichtigt, die seine Sicherheit gefährden würde. Durch Rückkopplung dieser erfassten und interpretierten Hirnaktivitäten auf eine Steuerung der Maschine kann eine Reaktion der Maschine ausgelöst werden, die eine bevorstehende Gefahrensituation abwendet. Beispielsweise erkennt das System, dass der Anwender beabsichtigt seine Hand in eine Säge zu stecken und veranlasst daher eine Abschaltung der Säge.

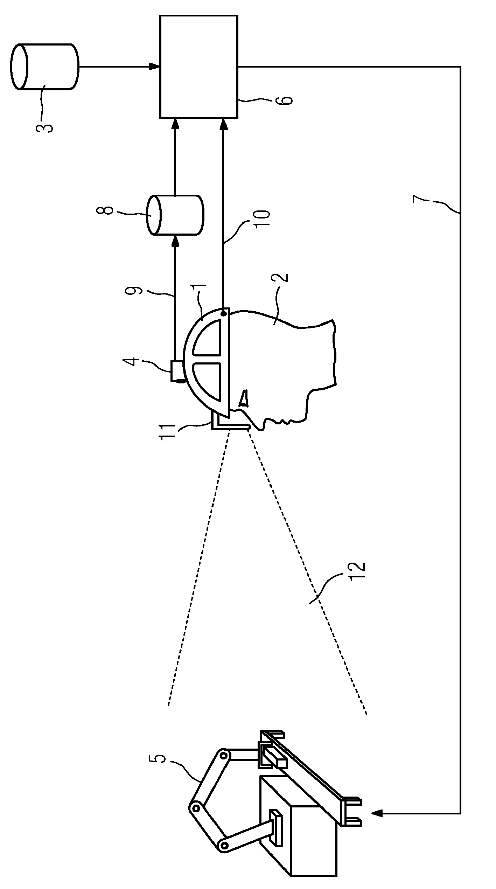

Im Folgenden wird die Erfindung anhand des in der Figur dargestellten Ausführungsbeispiels näher beschrieben und erläutert.

Die Figur zeigt ein Anwendungsszenario einer erfindungsgemäßen Ausführungsform des Systems in der Automatisierungstechnik.

Ein Anwender 2 trägt ein Messsystem 1 zur Erfassung seiner Hirnaktivitäten 10. Das Messsystem ist als EEG-Kappe ausgeführt, die der Anwender auf seinem Kopf trägt, und dient zur Messung der Hirnströme des Anwenders 2.

Ebenfalls auf dem Kopf des Anwenders 2 befindet sich ein Trackingsystem 4, das beispielsweise eine Stereokamera aufweist, mit der die Umgebung des Anwenders erfasst werden kann. Die Stereokamera ist zur Ermittlung eines aktuellen Wahrnehmungsbereiches 12 des Anwenders 2 vorgesehen.

In einem Speicher ist ein 3-dimensionales Modell abgelegt, welches die Umgebung des Anwenders 2 beschreibt. Mit Hilfe des Modells und des Trackingsystems 4 können die Objekte 5 und/oder Szenarien identifiziert werden, die sich im aktuellen Wahrnehmungsbereich 12 des Anwenders 2 befinden. In dem dargestellten Beispiel befindet sich ein Automatisierungssystem bestehend aus einem Positionierroboter und einem Förderband im Wahrnehmungsbereich 12 des Anwenders 2.

Durch die parallele Messung der Hirnaktivitäten des Anwenders 2 mit dem Messsystem 2 ist es möglich, eine Zuordnung der Hirnaktivitäten 10 zu dem identifizierten Objekt 5 oder Szenario herzustellen. Die Zuordnung geschieht mittels einer Verarbeitungseinheit 6.

Um die Ermittlung des Anwenderinteresses zu erleichtern, ist eine als Ringpuffer ausgeführte Aufzeichnungseinheit 8 vorgesehen, mit der die von dem Trackingsystem 4 ermittelten Informationen aufgezeichnet werden. Der Ringpuffer 8 enthält stets die Trackinginformationen der letzten 2 Sekunden.

Das System weist zusätzlich eine Datenbrille 11 auf, mit der dem Anwender 2 kontextsensitiv Informationen in sein Sichtfeld eingeblendet werden. Eine derartige Datenbrille 11 mit der beschriebenen Funktion ist aus Augmented Reality Systemen bekannt. Entsprechend des Szenarios im Wahrnehmungsbereich 12 des Anwenders 2 werden dem Anwender 2 bestimmte Informationen eingeblendet. Beispielsweise kann es sich hierbei um Serviceinformationen handeln, die der Anwender 2 zur Wartung des Positionierroboters benötigt.

Bei der Beobachtung des Positionierroboters am Förderband können bei dem Anwender 2 durch verschiedene Szenarien bestimmte Reize ausgelöst werden, die von dem System erkannt und für eine Steuerung des Förderbandes oder des Positionierroboters nutzbar gemacht werden können. Beobachtet der Anwender 2 einen Fehler in dem Automatisierungsprozess, wird dies von dem Messsystem zur Messung der Hirnaktivitäten erkannt. Das Trackingsystem 4 identifiziert mit Hilfe des im Speicher 3 abgelegten 3D-Modells der Umgebung den Positionierroboter und das Förderband als Fokus des Anwenderinteresses. Aus diesen beiden Informationen kann die Verarbeitungseinheit die Information generieren, das ein Fehler aufgetreten ist, der dem Positionierroboter bzw. dem Förderband zuzuordnen ist. Die Verarbeitungseinheit 6 generiert eine entsprechende Datenmenge 7 zur Beschreibung dieser Informationen, die z. B. über Funk an eine Signalverarbeitungseinrichtung gesendet wird, die dem aus dem Förderband und dem Positionierroboter bestehenden Automatisierungssystem 5 zugeordnet ist. Die Signalverarbeitungseinrichtung veranlasst je nach Art des Fehlers u. U. daraufhin eine Notabschaltung des Automatisierungssystems 5.

Zusammenfassend betrifft die Erfindung ein System und ein Verfahren zur Erfassung und Bewertung menschlicher Gehirnaktivitäten eines Anwenders im Zusammenhang einer Umgebung des Anwenders. Mit einem Trackingsystem wird eine aktuelle Position des Anwenders in Bezug auf seine Umgebung erfasst. Mit einem Umgebungsmodell wird die reale Umgebung des Anwenders soweit modelliert, dass das System Objekte und Szenarien im Umfeld des Anwenders erkennen kann. Die Hirnaktivitäten werden gemessen und den identifizierten realen Objekten bzw. Szenarien zugeordnet, so dass das System eine Korrelation zwischen der den Anwender aktuell umgebenden Realität und seinen Hirnaktivitäten herstellen kann.

## Patentansprüche

1. System mit
• einem Messsystem (1) zur Erfassung von Hirnaktivitäten (10) eines Anwenders (2),
• einem Speicher (3) mit einem Umgebungsmodell einer realen Umgebung des Anwenders (2),
• einem Trackingsystem (4) zur Ermittlung von in einem aktuellen Wahrnehmungsbereich (12) des Anwenders befindlichen Objekten (5) und/oder Szenarien in der Umgebung des Anwenders (2), wobei eine Identifikation der Objekte (5) und/oder Szenarien auf Basis des Umgebungsmodells vorgesehen ist,
• und mit einer Verarbeitungseinheit (6) zur Interpretation der Hirnaktivitäten (10) auf Basis einer Korrelation der Hirnaktivitäten (10) mit den im aktuellen Wahrnehmungsbereich (12) des Anwenders befindlichen Objekten (5) und/oder Szenarien.

2. System nach Anspruch 1,
**dadurch gekennzeichnet, dass** die Verarbeitungseinheit (6) zur Erzeugung einer Datenmenge (7) vorgesehen ist, die das Ergebnis der Interpretation beschreibt.

3. System nach Anspruch 2 mit Sendemitteln zur insbesondere drahtlosen Übertragung der Datenmenge (7) an das Objekt (5), wobei das Objekt als technische Vorrichtung mit einer Signalverarbeitungseinheit zur Verarbeitung der Datenmenge (7) ausgebildet ist.

4. System nach einem der vorhergehenden Ansprüche,
**dadurch gekennzeichnet, dass** das Trackingsystem (4) zur Bestimmung der Position des Anwenders (2) in Bezug auf die Umgebung des Anwenders (2) vorgesehen ist.

5. System nach einem der vorhergehenden Ansprüche,
**dadurch gekennzeichnet, dass** das Trackingsystem (4) zur Bestimmung des Blickwinkels des Anwenders (2) in Bezug auf die Umgebung des Anwenders (2) vorgesehen ist.

6. System nach einem der vorhergehenden Ansprüche,
**dadurch gekennzeichnet, dass** das Trackingsystem (4) zur Lagebestimmung eines Körperteils des Anwenders (2) vorgesehen ist.

7. System nach einem der vorhergehenden Ansprüche mit einer Aufzeichnungseinheit (8) zur Aufzeichnung von durch das Trackingsystem (4) ermittelten Informationen (9).

8. System nach einem der vorhergehenden Ansprüche mit einem Augmented Reality System.

9. System nach einem der vorhergehenden Ansprüche mit einem Leitfähigkeitsmesssystem zur Bestimmung der Leitfähigkeit der Haut des Anwenders (2).

10. System nach einem der vorhergehenden Ansprüche,
**dadurch gekennzeichnet, dass** das Messsystem (1) zur Erfassung der Hirnaktivitäten (10) zur Hirnstrommessung vorgesehen ist.

11. System nach einem der vorhergehenden Ansprüche,
**dadurch gekennzeichnet, dass** das Messsystem (1) zur Erfassung der Hirnaktivitäten (10) zur Pupillenmessung vorgesehen ist.

12. Verwendung eines Systems nach einem der vorhergehenden Ansprüche in der Automatisierungstechnik.

13. Verwendung eines Systems nach einem der vorhergehenden Ansprüche zur Qualitätskontrolle in einem industriellen Fertigungsprozess.

14. Verwendung eines Systems nach einem der vorhergehenden Ansprüche zur Aufmerksamkeitskontrolle des Anwenders.

15. Verwendung eines Systems nach einem der vorhergehenden Ansprüche zur Ermittlung beabsichtigter Anwenderhandlungen an einem technischen System.

16. Verfahren, bei dem mit
• einem Messsystem (1) Hirnaktivitäten (10) eines Anwenders (2) erfasst werden,
• einem Trackingsystem (4) in einem aktuellen Wahrnehmungsbereich (12) des Anwenders (2) befindliche Objekte (5) und/oder Szenarien in der Umgebung des Anwenders (2) ermittelt werden, wobei die Objekte (5) und/oder Szenarien auf Basis eines Umgebungsmodells identifiziert werden,
• und bei dem mit einer Verarbeitungseinheit (6) die Hirnaktivitäten (10) auf Basis einer Korrelation der Hirnaktivitäten (10) mit den im aktuellen Wahrnehmungsbereich (12) des Anwenders befindlichen Objekten (5) und/oder Szenarien interpretiert werden.

17. Verfahren nach Anspruch 16,
**dadurch gekennzeichnet, dass** mit der Verarbeitungseinheit (6) eine Datenmenge (7) erzeugt wird, die das Ergebnis der Interpretation beschreibt.

18. Verfahren nach Anspruch 17,
**dadurch gekennzeichnet, dass** die Datenmenge (7) insbesondere drahtlos an das Objekt (5) gesendet wird, wobei das Objekt als technische Vorrichtung mit einer Signalverarbeitungseinheit zur Verarbeitung der Datenmenge (7) ausgebildet ist.

19. Verfahren nach einem der Ansprüche 16 bis 18,
**dadurch gekennzeichnet, dass** mit dem Trackingsystem (4) die Position des Anwenders (2) in Bezug auf die Umgebung des Anwenders (2) bestimmt wird.

20. Verfahren nach einem der Ansprüche 16 bis 19,
**dadurch gekennzeichnet, dass** mit dem Trackingsystem (4) der Blickwinkels des Anwenders (2) in Bezug auf die Umgebung des Anwenders (2) bestimmt wird.

21. Verfahren nach einem der Ansprüche 16 bis 20,
**dadurch gekennzeichnet, dass** mit dem Trackingsystem (4) die Lage eines Körperteils des Anwenders (2) bestimmt wird.

22. Verfahren nach einem der Ansprüche 16 bis 21,
**dadurch gekennzeichnet, dass** mit einer Aufzeichnungseinheit (8) durch das Trackingsystem (4) ermittelte Informationen (9) aufgezeichnet werden.

23. Verfahren nach einem der Ansprüche 16 bis 22,
**dadurch gekennzeichnet, dass** dem Anwender (2) Augmented Reality Informationen mit Hilfe des Systems visualisiert werden.

24. Verfahren nach einem der Ansprüche 16 bis 23,
**dadurch gekennzeichnet, dass** mit einem Leitfähigkeitsmesssystem die Leitfähigkeit der Haut des Anwenders (2) bestimmt wird.

25. Verfahren nach einem der Ansprüche 16 bis 24,
**dadurch gekennzeichnet, dass** mit dem Messsystem (1) die Hirnströme des Anwenders (2) gemessen werden.

26. Verfahren nach einem der Ansprüche 16 bis 25,
**dadurch gekennzeichnet, dass** mit dem Messsystem (1) die Pupillenweite des Anwenders (2) gemessen wird.
